# EUROPEAN PATENT APPLICATION

(11) **EP 1 555 035 A2**
(43) Date of publication of application: **20.07.2005**
(21) Application number: 04258168.6
(22) Date of filing: 29.12.2004
(51) Int. Cl.: A61L 27/48, A61L 27/56, A61L 27/58

(54) **Collagen matrix for soft tissue augmentation**

(30) Priority: 30.12.2003 US 748894
(71) Applicant: ETHICON ENDO-SURGERY, INC., Cincinnati, OH 45242-2839 (US)
(72) Inventor: Freeman, Lynetta, West Chester, Ohio 45069 (US); Roweton, Susan, Raleigh, North Carolina 27614 (US); Walthall, Ben, Whitehouse Station, New Jersey 08889 (US); Nguyen, Kien, Doylestown, Pennsylvania 18901 (US)
(74) Representative: Tunstall, Christopher Stephen

(57) **Abstract**

The present invention includes methods and materials for soft tissue implant formed from biologically-compatible polymeric matrixes. The matrixes may have pores sized for ingrowth of soft tissue. The material may be utilized with collagen or other matrix materials. This material may be used in a method of reforming soft tissues by implanting the material within soft body tissues to modify soft tissue defects such as wrinkles or biopsy tissue defects and to reshape soft tissue.

## Description

### FIELD OF THE INVENTION

The present invention relates to reformation of soft tissues within the body. More particularly, the invention relates to compositions useful in reforming the shape of soft tissues and methods of using such compositions in reforming soft tissues.

### BACKGROUND OF THE INVENTION

Breast cancer is one of the leading causes of cancer death among women. Early detection and treatment greatly increase long-term survival rates, but many women delay seeking treatment of suspected lesions out of fear of mutilation. (Ersek RA, Denton DR. Breast biopsy technique: A plea for cosmesis. Southern Medical Journal 79:167-170, 1986.) Each year, more than 1 million women receive breast biopsy procedures. The fear is compounded by the fact that although approximately 80% of all biopsies prove to be benign, many women are left with unsightly scarring and contour defects from the biopsies themselves. Because of the psychological trauma involved in the diagnosis and treatment of breast cancer and the importance of early detection to long-term survival, surgeons are interested in minimizing the potential for disfigurement. In breast cancer patients, cosmetic outcome has correlated closely with the psychological and physical well-being of the patient. (Yeo W, Kwan WH, Teo PML, et al: Cosmetic outcome of breast-conserving therapy in Chinese patients with early breast cancer. Aust NZJ Surg 67:771-771, 1997.)

Though a certain amount of disfigurement is unavoidable in many cases, subsequent plastic surgery may be able to restore much of the original appearance. However, many women are reluctant to undergo the trauma and time of additional surgical procedures. Alternatives to improve cosmesis include injection of liquids, transplantation of free or vascularized fat and/or muscle flaps and grafts, and the use of silicone or saline implants. Unfortunately, implant approaches are complicated by the body forming hard fibrous capsules around the implanted materials, which contracts over time, causing breast pain.

The medical community for many years has been attempting to develop materials and techniques to replace tissues with the body. It may be desirable to replace such tissue due to, for example, injury, disease, side effects of medical procedures and surgeries, and the aging process, for example. In addition, some patients may desire to alter their appearance for cosmetic reasons, particularly the *contour* of visible soft tissues. Much attention has been given to the reformation of soft tissue to locally increase its volume and change its shape.

Numerous replacement materials have been tried, with certain advantages and disadvantages. Silicone has been used but can displace and harden over time. Plastic and metal implants have also been used. However, implants such as these may not have a "natural" look or feel, especially as the body changes over time.

Accordingly, it would be desirable to have substitute materials for soft tissues. It would also be desirable to have a soft tissue replacement material that was supple, flexible, and durable. Also, a replacement material that could be implanted in with minimal effort for greater ease during surgical procedures would be highly desirable.

### SUMMARY OF THE INVENTION

The invention features devices and methods for soft tissue substitute. The present invention features materials that may be implanted into soft body tissue for correction of soft tissue defects or for soft tissue augmentation. The material comprises a biologically-compatible lattice or matrix. The material may be combined with collagen, proteins or other matrix materials.

Preferably, the matrix is absorbable, biocompatible, used as an acellular or cellular substrate, supports native tissue in-growth in three dimensions, and maintains the biomechanical properties of tissue (e.g., breast tissue) during the postoperative period. The highly porous nature of this product serves as a support structure into which vessels and mesenchymal cells from the wound site migrate. The cells and vessels create new tissue that replaces the collagen glycosaminoglycan as it biodegrades. Because cells invade the matrix material, fibrous encapsulation does not occur to any great degree.

In addition, cells may be seeded on the lattice substrate. Such cells may be adipocytes (fat cells) and/or preadipocytes (fat cell precursors that differentiate into adipocytes) derived from fat storage areas in the body or fat cells derived from bone marrow, stem cells, or mesenchymal cells that are harvested and developed under laboratory conditions and seeded onto the matrix prior to implantation.

Preferably, the collagen glycosaminoglycan material is placed directly into the wound following surgery to fill the cavity and correct contour defects resulting from the removal of tissue. The product may be used at the time of the initial surgery, or in subsequent surgery.

The material used in this invention is preferably in sheet form. The sheet may be randomly folded, rolled, cinched, or configured otherwise to fill the defect. Other forms of the collagen glycosaminoglycan include forms such as blocks (e.g., 2.5 cm x 2.5 cm x 1 cm blocks), spheres, and other configurations.

It is an object of the present invention to provide a soft tissue substitute. It is another object of the invention to provide a soft tissue substitute that is at least partially non-resorbable, supple, flexible and durable so that patients do not need to undergo repeated procedures.

Another object of the invention is to minimize patient discomfort, risk of infection and side effects of repeated medical procedures.

It is another object of this invention to provide a soft tissue substitute that may be implanted into the body and does not migrate.

It is a further object of this invention to provide a soft tissue substitute that is synthetic, bioinert and may contain natural materials. It is yet another object of the invention to provide a soft tissue substitute that may be used to reform and augment soft tissues, including soft tissue contour defects. In addition, the implant material may incorporate radio-opaque materials.

The present invention is a soft tissue implant material comprising biologically-compatible polymeric matrix. The matrix may have a porous surface. The implant material may be combined with a variety of matrix materials, including collagen. The implant material may also contain biologically active substances, which may, for example, be bound to the matrix. The implant material may be formed by known methods.

The invention also features methods for reforming and augmenting soft tissues. The implant material may be implanted into soft tissue at a desired location. It may be accurately placed within soft tissue using a hand or orthoscopic device. In this manner, the implant material may be used to correct soft tissue defects, (*e*.*g*. by plumping and expanding tissues).

The present invention pertains to a method for correcting contour defects within a human or animal which comprises the steps of applying a lattice sheet, e.g., a collagen-glycosaminoglycan matrix (CG matrix), within the surgical cavity. Blood vessels and mesenchymal cells are allowed to infiltrate the CG matrix from tissue within the cavity of a subject.

One of the preferred lattices is a synthetic membrane (hereinafter referred to as "CG matrix") which is a highly porous lattice made of collagen and glycosaminoglycan. The CG lattice serves as a supporting or scaffolding structure into which blood vessels and mesenchymal cells migrate from within a tissue cavity, a process referred to as "infiltration". Infiltration is responsible for creating a new tissue, which replaces the CG matrix as it biodegrades.

Various forms of glycosaminoglycans that may be suitable for use in this material include chondroitin 6-sulfate, chondroitin 4-sulfate, heparin, heparin sulfate, keratan sulfate, dermatan sulfate, chitin and chitosan.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

Throughout this document, all temperatures are given in degrees Celsius, and all percentages are weight percentages unless otherwise stated. All publications mentioned herein are incorporated herein by reference for the purpose of describing and disclosing the compositions and methodologies which are described in the publications which might be used in connection with the presently described invention. The publications discussed herein are provided solely for their disclosure prior to the filing date of the present application. Nothing herein is to be construed as an admission that the invention is not entitled to antedate such a disclosure by virtue of prior invention.

### BRIEF DESCRIPTION OF THE DRAWINGS

This invention, as defined in the claims, can be better understood with reference to the following drawings. The drawings are not necessarily to scale, emphasis instead being placed upon clearly illustrating principles of the present invention.

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

FIG. 1 shows a schematic of the steps to the method of contour defect repair. In FIG. 1A, a surgical biopsy or lumpectomy procedure leaves a cavity in the tissue. Following the surgical biopsy or lumpectomy procedure, the wound cavity is inspected to ensure appropriate hemostasis. In FIG. 1B, the material is randomly packed into the cavity site. If FIG. 1C, the subcutaneous tissue and skin incisions are closed routinely.

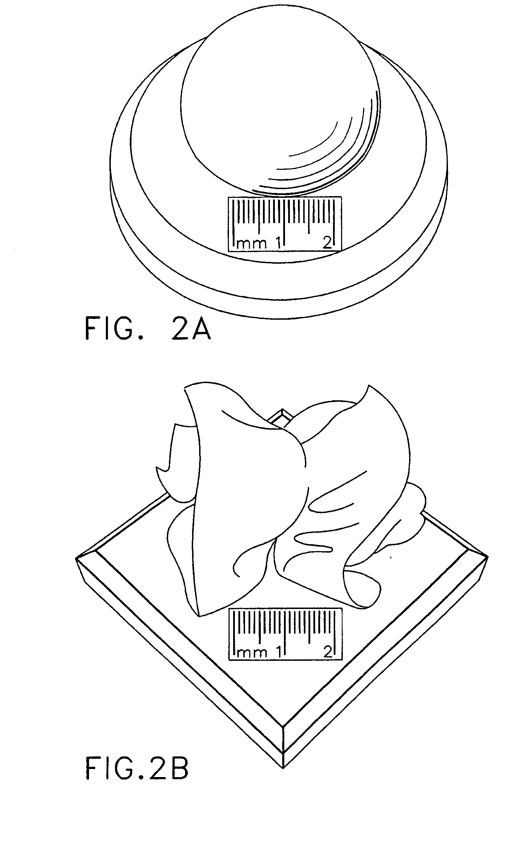

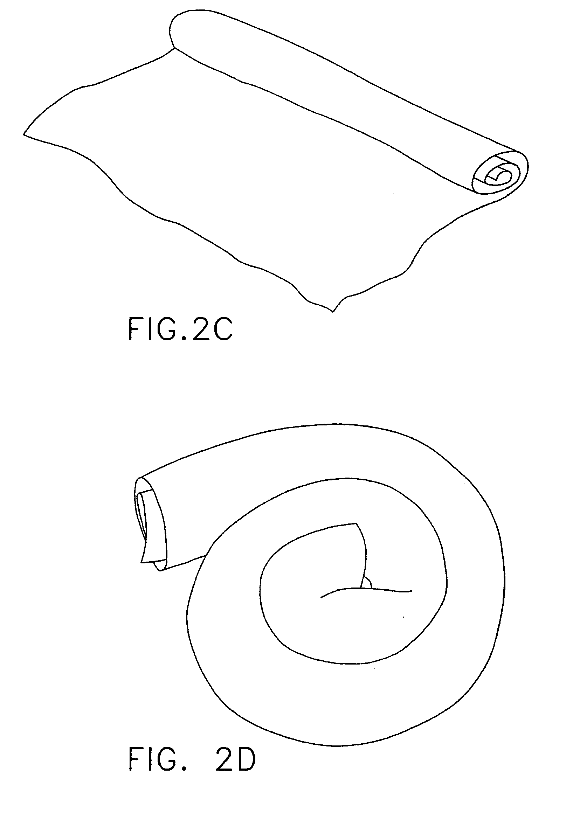

FIG. 2 shows various forms of the lattice device of the present invention as a ball (2A), sheet (2B), coil (2C), and roll (2D).

In the following description of the illustrated embodiments, references are made to the accompanying drawings, which form a part hereof, and in which is shown by way of illustration various embodiments in which the invention may be practiced. It is to be understood that other embodiments may be utilized, and structural and functional changes may be made without departing from the scope of the present invention.

### DETAILED DESCRIPTION OF THE PREFERRED EMBODIMENT

Before the present device and methods for tissue augmentation is described, it is to be understood that this invention is not limited to the specific methodology, devices, formulations, and surgical defects described as such may, of course, vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "and", and "the" include plural referents unless the context clearly dictates otherwise. Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

The invention features devices and methods for making and using a permanent implant. The present invention refers to a method of correcting contour defects on a human or animal. This invention overcomes many of the shortcomings presented by methods presently used to fill defects by using a preformed lattice structure for insertion into a surgical cavity.

The invention features devices and methods for soft tissue substitute. The present invention features materials that may be implanted into soft body tissue for correction of soft tissue defects or for soft tissue augmentation. The material comprises a biologically-compatible lattice or matrix. The material may be combined with collagen, proteins or other matrix materials.

Preferably, the matrix is absorbable, biocompatible, used as an acellular or cellular substrate, supports native tissue in-growth in three dimensions, and maintains the biomechanical properties of tissue (*e.g.*, breast tissue) during the postoperative period. The highly porous nature of this product serves as a support structure into which vessels and mesenchymal cells from the wound site migrate. The cells and vessels create new tissue that replaces the collagen glycosaminoglycan as it biodegrades. Because cells invade the matrix material, fibrous encapsulation does not occur to any great degree.

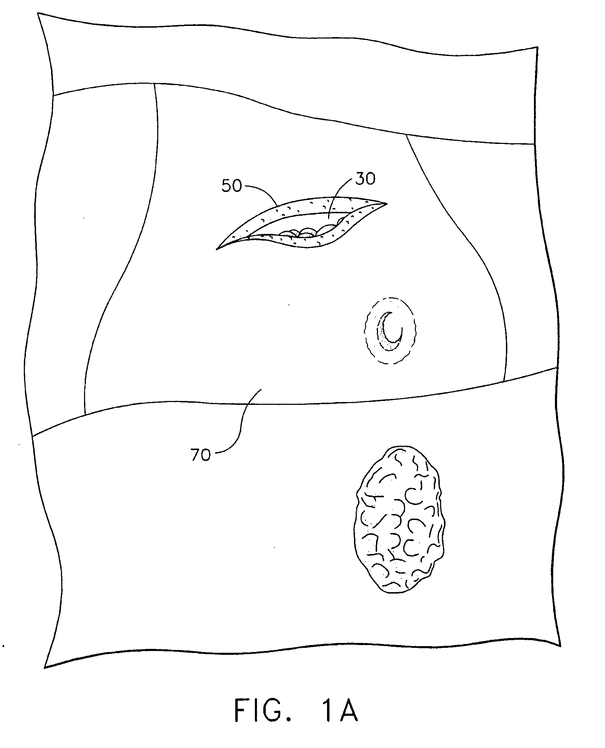

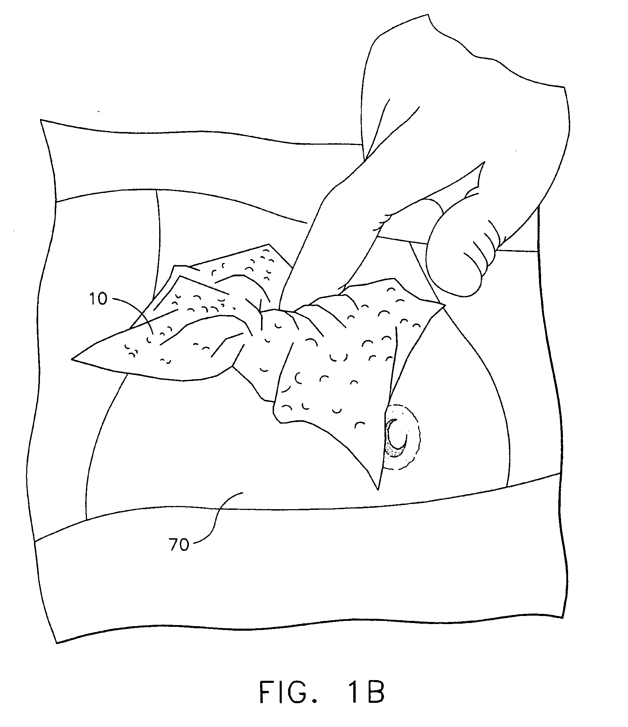

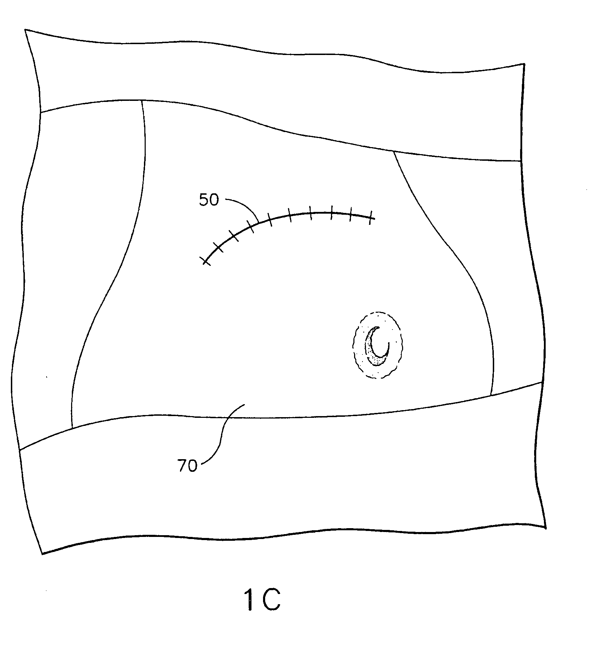

As shown in FIG. 1A, a surgical biopsy or lumpectomy procedure is performed through an opening 50 in the tissue 70 of a patient that leaves a cavity 30 in the tissue 70. Following the surgical biopsy or lumpectomy procedure, the cavity 30 is inspected to ensure appropriate hemostasis. As shown in FIG. 1B, the lattice device 10 is randomly packed into the cavity 30 through the opening 50. As shown in FIG. 1C, the opening 50 in the subcutaneous tissue and skin 70 is closed using routine surgical procedures known in the art.

Preferably, the device 10 comprising a lattice or matrix comprises a synthetic material having at least one layer. Preferably, at least one layer of the matrix comprises a protein. More preferably, the protein is selected from the group consisting of fibrin, collagen, glycosaminoglycan, and combinations thereof. Generally, the device is a soft tissue implant material comprising biologically-compatible polymeric lattice having pores, said pores having dimensions effective to permit soft tissue to grow therein. Preferably, the pores comprise between about zero and about 90 percent of said implant material. More preferably, the pores comprise between about 20 and about 80 percent of said implant material. More preferably, the pores comprise between about 40 and about 60 percent of said implant material. Generally, the pores have a size of less than about 100 microns. Preferably, the implant material is constructed of collagen comprising between about 30% and about 65% of the implant material by volume. In another embodiment, the lattice has an overall thickness of from about 10 to about 200 mils, preferably 25 to about 100 mils.

Therefore, in one embodiment, the invention provides for a method of augmenting soft tissue comprising: (a) providing a biologically-compatible implant material comprised of biologically compatible polymeric lattice; and (b) implanting said implant material within a cavity 30 within soft tissue 70. Preferably, the lattice is in the form of a sheet. Preferably, the implanting step includes inserting the implant material subcutaneously into an area having a soft tissue contour defect in an amount sufficient to at least partially remove the contour defect.

In one embodiment, the lattice is formed from a crosslinked collagen-glycosaminoglycan composite containing at least about 0.5% by weight glycosaminoglycan. In another embodiment, the collagen-glycosaminoglycan composite contains a sulfate-containing glycosaminoglycan. In another embodiment, the collagen-glycosaminoglycan composite contains from about 6% to about 12% by weight of said sulfate-containing glycosaminoglycan. In another embodiment, the sulfate-containing glycosaminoglycan is selected from chondroitin 6-sulfate, chondroitin 4-sulfate, heparin, heparan sulfate, keratan sulfate or dermatan sulfate. In another embodiment, the collagen-glycosaminoglycan composite is crosslinked to an Mc value of between about 800 and about 60,000. Preferably, the sulfate-containing glycosaminoglycan is chondroitin 6-sulfate.

Preferably, the collagen glycosaminoglycan material is placed directly into the wound following surgery to fill the cavity and correct contour defects resulting from the removal of tissue. The product may be used at the time of the initial surgery, or in subsequent surgery.

The material used in this invention is preferably in sheet form. The sheet may be randomly folded, rolled, cinched, or configured otherwise to fill the defect. Other forms of the collagen glycosaminoglycan include forms such as blocks (*e*.*g*., 2.5 cm x 2.5 cm x 1 cm blocks), spheres, and other configurations. Optionally, the sheet or other form may be cut to the size required to fill the defect.

The present invention is a soft tissue implant material comprising biologically-compatible polymeric matrix. The matrix may have a porous surface. The implant material may be combined with a variety of matrix materials, including collagen. The implant material may also contain bioactive substances, which may, for example, be grafted to the matrix. The implant material may be formed by known methods.

Generally, the bioactive compounds of the invention are administered in a therapeutically effective amount, *i*.*e*., a dosage sufficient to effect treatment, which may vary depending on the individual and condition being treated. By way of example only, a therapeutically effective daily dose may be from 0.1 to 100 mg/kg of body weight per day of drug. Many conditions may respond to administration of a total dosage of between about 1 and about 30 mg/kg of body weight per day, or between about 70 mg and 2100 mg per day for a 70 kg person. Other dosages may be administered without departure from the present invention.

The devices of the present invention may also be used for localized delivery of various drugs and other biologically active agents. Biologically active agents such as growth factors may be delivered from the device to a local tissue site in order to facilitate tissue healing and regeneration.

The term "biologically active agent" or "active agent" as used herein refers to organic molecules that exert biological effects in vivo. Examples of active agents include, without limitation, enzymes, receptor antagonists or agonists, hormones, growth factors, angiogenic factors, autogenous bone marrow, antibiotics, antimicrobial agents and antibodies. The term "active agent" is also intended to encompass various cell types and genes that can be incorporated into the devices of the invention. The term "active agent" is also intended to encompass combinations or mixtures of two or more active agents, as defined above.

Preferred growth factors include transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors. Members of the transforming growth factor (TGF) supergene family, which are multifunctional regulatory proteins, are particularly preferred. Members of the TGF supergene family include the beta transforming growth factors (for example, TGF-β1, TGF-β2, TGF-β3); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); inhibins (for example, Inhibin A, Inhibin B); growth differentiating factors (for example, GDF-1); and Activins (for example, Activin A, Activin B, Activin AB).

Steroidal anti-inflammatories can be used to decrease inflammation to the implanted device. These factors are known to those skilled in the art and are available commercially or described in the literature.

Preferably, the active agents are incorporated to between 1 % and 30% by weight, although the factors can be incorporated to a weight percentage between 0.01 and 95 weight percentage. Active agents can be incorporated into the device and released over time by diffusion and/or degradation of the device or incorporated within the device, or some combination thereof. The type and amount of active agent used will depend, among other factors, on the particular site and condition to be treated and the biological activity and pharmacokinetics of the active agent selected.

Cytotoxic and immunosuppressive drugs may constitute an additional class of drugs for which the implant devices of the invention may be useful. These agents are commonly used to treat hyperproliferative diseases such as psoriasis, as well as for immune diseases such as bullous dermatoses and leukocytoclastic vasculitis. Examples of such compounds include, but are not limited to, antimetabolites such as methotrexate, azathioprine, fluorouracil, hydroxyurea, 6-thioquanine, mycophenolate, chlorambucil, vinicristine, vinblasrine and dactinomycin. Other examples include alkylating agents such as cyclophosphamide, mechloroethamine hydrochloride, carmustine, taxol, tacrolimus and vinblastine are additional examples of useful biological agents, as are dapsone and sulfasalazine. Ascomycins, such as Cyclosporine, FK506 (tacrolimus), and rapamycin (e.g., U.S. Pat. No. 5,912,253) and analogs of such compounds are of particular interest (*e.g.*, Mollinson *et al*., Current Pharm. Design 4(5):367-380 (1998); U.S. Pat. Nos. 5,612,350; 5,599,927; 5,604,294; 5,990,131; 5,561,140; 5,859,031; 5,925,649; 5,994,299; 6,004,973 and 5,508,397). Cyclosporins include cyclosporin A, B, C, D, G and M. See, *e*.*g*., U.S. Pat. Nos. 6,007,840; and 6,004,973. Another aspect of the invention comprises delivery of taxane- and taxoid anticancer compositions, which are particularly useful for inhibiting growth of cancer cells.

The implant devices may include bioactive agents useful for treating conditions such as lupus erythematosus (both discoid and systemic), cutaneous dermatomyositis, porphyria cutanea tarda and polymorphous light eruption. Agents useful for treating such conditions include, for example, quinine, chloroquine, hydroxychloroquine, and quinacrine.

The implant devices of the invention may also useful for transdermal delivery of antiinfective agents. For example, antibacterial, antifungal and antiviral agents may be used with the implant devices. Antibacterial agents may be useful for treating conditions such as acne, cutaneous infections, and the like. Antifungal agents may be used to treat tinea corporis, tinea pedis, onychomycosis, candidiasis, tinea versicolor, and the like. Examples of antifungal agents include, but are not limited to, azole antifungals such as itraconazole, myconazole and fluconazole. Examples of antiviral agents include, but are not limited to, acyclovir, famciclovir, and valacyclovir. Such agents may be useful for treating viral diseases, e.g., herpes.

Another example of a bioactive agent for which the implant devices of the invention may include are the antihistamines. These agents are useful for treating conditions such as pruritus due to urticaria, atopic dermatitis, contact dermatitis, psoriasis, and many others. Examples of such reagents include, for example, terfenadine, astemizole, lorotadine, cetirizine, acrivastine, temelastine, cimetidine, ranitidine, famotidine, nizatidine, and the like. Tricyclic antidepressants may also be delivered.

Pain relief agents and local anesthetics constitute another class of compounds for which the implant devices of the invention may enhance treatment. Lidocaine, bupibacaine, novocaine, procaine, tetracaine, benzocaine, cocaine, and the opiates, are among the compounds that may be used with the implant devices of the invention.

In one embodiment, the bioactive agent formulation comprises a cardiac drug including, but is not limited to: angiogenic factors, growth factors, calcium channel blockers, antihypertensive agents, inotropic agents, antiatherogenic agents, anti-coagulants, beta-blockers, anti-arrhythmic agents, anti-inflammatory agents, sympathomimetic agents, phosphodiesterase inhibitors, diuretics, vasodilators, thrombolytic agents, cardiac glycosides, antibiotics, antiviral agents, antifungal agents, agents that inhibit protozoans, antineoplastic agents, and steroids.

The term "anti-arrhythmia agent" or "anti-arrhythmic" refers to any drug used to treat a disorder of rate, rhythm or conduction of electrical impulses within the heart. The term "angiogenic agent" (or "angiogenic factor") means any compdund that promotes growth of new blood vessels. Angiogenic factors include, but are not limited to, a fibroblast growth factor, e.g., basic fibroblast growth factor (bFGF), and acidic fibroblast growth factor, e.g., FGF-1, FGF-2, FGF-3, FGF-4, recombinant human FGF (U.S. Pat. No. 5,604,293); a vascular endothelial cell growth factor (VEGF), including, but not limited to, VEGF-1, VEGF-2, VEGF-D (U.S. Pat. No. 6,235,713); transforming growth factor-alpha; transforming growth factor-beta; platelet derived growth factor; an endothelial mitogenic growth factor; platelet activating factor; tumor necrosis factor-alpha; angiogenin; a prostaglandin, including, but not limited to PGE1, PGE2; placental growth factor; GCSF (granulocyte colony stimulating factor); HGF (hepatocyte growth factor); IL-8; vascular permeability factor; epidermal growth factor; substance P; bradykinin; angiogenin; angiotensin II; proliferin; insulin like growth factor-1; nicotinamide; a stimulator of nitric oxide synthase; estrogen, including, but not limited to, estradiol (E2), estriol (E3), and 17-beta estradiol; and the like.

Angiogenic factors further include functional analogs and derivatives of any of the aforementioned angiogenic factors. Derivatives include polypeptide angiogenic factors having an amino acid sequence that differs from the native or wild-type amino acid sequence, including conservative amino acid differences (e.g., serine/threonine, asparagine/glutamine, alanine/valine, leucine/isoleucine, phenylalanine/tryptophan, lysine/arginine, aspartic acid/glutamic acid substitutions); truncations; insertions; deletions; and the like, that do not substantially adversely affect, and that may increase, the angiogenic property of the angiogenic factor. Angiogenic factors include factors modified by polyethylene glycol modifications; acylation; acetylation; glycosylation; and the like. An angiogenic factor may also be a polynucleotide that encodes the polypeptide angiogenic factor. Such a polynucleotide may be a naked polynucleotide or may be incorporated into a vector, such as a viral vector system such as an adenovirus, adeno-associated virus or lentivirus systems.

Antibiotics are among the bioactive agents that may be useful when used with the implant devices of the invention, particularly those that act on invasive bacteria, such as Shigella, Salmonella, and Yersinia. Such compounds include, for example, norfloxacin, ciprofloxacin, trimethoprim, sulfamethyloxazole, and the like. Anti-neoplastic agents may also be delivered by the implant devices of the invention including, for example, cisplatin, methotrexate, taxol, fluorouracil, mercaptopurine, donorubicin, bleomycin, and the like.

Exemplary anti-inflammatory agents include, but are in no way limited to, corticoids such as cortisone and ACTH, dexamethasone, cortisol, interleukin-1 and its receptor antagonists, and antibodies to TGF-beta, to interleukin-1 (IL-1), and to interferon-gamma. Exemplary anti-oxidants include, but are in no way limited to, vitamin C (ascorbic acid) and vitamin E. Exemplary angiogenic factors include, but are in no way limited to, fibroblast growth factor and nerve growth factor.

Angiogenic growth factors which may be used in the device include, but are not limited to, Basic Fibroblast Growth Factor (bFGF), (also known as Heparin Binding Growth Factor-II and Fibroblast Growth Factor II), Acidic Fibroblast Growth Factor (aFGF), (also known as Heparin Binding Growth Factor-I and Fibroblast Growth Factor-I), Vascular Endothelial Growth Factor (VEGF), Platelet Derived Endothelial Cell Growth Factor BB (PDEGF-BB), Angiopoietin-1, Transforming Growth Factor Beta (TGF-Beta), Transforming Growth Factor Alpha (TGF-Alpha), Hepatocyte Growth Factor, Tumor Necrosis Factor-Alpha (TNF-Alpha), Angiogenin, Interleukin-8 (IL-8), Hypoxia Inducible Factor-I (H1F-1), Angiotensin-Converting Enzyme (ACE) Inhibitor Quinaprilat, Angiotropin, Thrombospondin, Peptide KGHK, Low Oxygen Tension, Lactic Acid, Insulin, and Growth Hormone.

The invention also features methods for reforming and augmenting soft tissues. The implant material may be implanted into soft tissue at a desired location. In injectable form it may be accurately placed within soft tissue using a syringe or orthoscopic device. In this manner, the implant material may be used to correct soft tissue defects, (*e*.*g*. by plumping and expanding tissues) remediate medical conditions such as incontinence, and for cosmetic procedures.

The present invention provides for methods for correcting contour defects within a human or animal which comprises the steps of applying a lattice sheet within the surgical cavity. Blood vessels and tissue cells are allowed to infiltrate the lattice from tissue within the cavity of a subject. The present invention has many advantages. The lattice is preferably completely synthetic and biodegradable over time. There is therefore no risk of disease transmission from donor to patient. In addition, synthetic materials provide reproducible components and manufacturing methods.

In another embodiment, the lattice is made up of multiple layers wherein the layers'are constructed from either proteinaceous or synthetic materials, or a combination wherein at least one layer is constructed from a proteinaceous material and at least one layer is constructed from a synthetic material. The layers themselves can be constructed from all proteinaceous or all synthetic materials, or a combination of proteins and synthetic materials. Examples of suitable proteins include alginates, fibrin, collagen, and glycosaminoglycan. Examples of suitable synthetic materials include hydrogels, such as polyethylene glycol.

A preferred embodiment of the present invention employs a highly porous lattice comprised of collagen and glycosaminoglycan (referred to hereinafter as "GAG"), *i*.*e*. a collagen glycosaminoglycan matrix (referred to hereinafter as "CG matrix"). See U.S. Pat. Nos. 4,060,081, 4,280,954 and 4,505,266, the teachings of which are incorporated herein in their entirety. Various forms of GAG which may be suitable for use in this material include chondroitin 6-sulfate, chondroitin 4-sulfate, heparin, heparin sulfate, keratan sulfate, dermatan sulfate, chitin and chitosan. See also US 5489304A1, US 5997895A1, WO9913902A1, and WO9706837A1.

The CG lattice serves as a supporting or scaffolding structure into which blood vessels and surrounding tissue cells migrate from within a tissue cavity, a process referred to as "infiltration". Infiltration is responsible for creating a new tissue, which replaces the lattice as it biodegrades.

The term "lattice" is used broadly herein to include any material that is in the form of a highly porous and permeable structure in which cells can migrate and proliferate. "Fibrous lattices" should be construed broadly to include all lattices, which include material that is fibrous at the macroscopic, microscopic, or molecular level. For example, many polymeric foams comprise long organic molecules, which may have numerous side chains or extensive crosslinking. The lattice of the present invention is not limited to collagen. Other fibrous proteins, other polymeric molecules, or sintered ceramics may also be suitable for prosthetic or other medical purposes.

The lattice serves as a temporary substitute for the tissue and can be any structure that has the following characteristics: the composition and structure of the lattice must be such that is does not provoke a substantial immune response from the graft recipient; the lattice must be sufficiently porous to permit blood vessels and cells from healthy tissue to migrate into the lattice; the lattice is biodegradable and the biodegradation must not proceed so rapidly that the lattice disappears before sufficient healing occurs.

It is possible to control several parameters of the CG matrix (primarily crosslinking density, porosity and GAG content) to control the rate of biodegradation of the lattice. Specific conditions for forming a CG matrix suitable for use in the present invention are given below. However, the skilled artisan will know of other conditions for forming CG matrices with variations of the above-mentioned parameters that are similarly suitable for use in the present invention. In addition, certain applications of tissue regeneration may require matrices that degrade more slowly or more quickly. The skilled artisan will be able to recognize applications where it is desirable to vary the properties of the CG matrix, and will be able to vary the parameters accordingly. The present invention encompasses such variations in the CG matrix.

Collagen is a major protein constituent of connective tissues in vertebrate as well as invertebrate animals. It is often present in the form of macroscopic fibers that can be chemically and mechanically separated from non-collagenous tissue components. Collagen derived from any source is suitable for use with this invention, including insoluble collagen, collagen soluble in acid, in neutral or basic aqueous solutions, as well as those collagens, which are commercially available. Typical animal sources include calfskin, bovine Achilles tendon, cattle bones and rat tail tendon.

Several levels of structural organization exist in collagen. The primary structure consists of the complete sequence of amino acids. Collagen is made up of 18 amino acids in relative amounts, which are well known for several animal species but in sequences and which are still not completely determined. The total content of acidic, basic and hydroxylated amino acid residues far exceeds the content of lipophilic residues making collagen a hydrophilic protein. Because of this, polar solvents with high solubility parameters are good solvents for collagen.

The term mucopolysaccharide describes hexosamine-containing polysaccharides of animal origin. Another name often used for this class of compounds is glycosaminoglycans. Chemically, mucopolysaccharides are alternating copolymers made up of residues of hexosamine glycosidically bound and alternating in a more-or-less regular manner with either hexuronic acid or hexose moieties.

Typical sources of heparin include hog intestine, beef lung, bovine liver capsule and mouse skin. Hyaluronic acid can be derived from rooster comb and human umbilical cord, whereas both chondroitin 4-sulfate and chondroitin 6-sulfate can be derived from bovine cartilage and shark cartilage. Dermatan sulfate and heparan sulfate can be derived from hog mucosal tissues while keratan sulfate can be derived from the bovine cornea.

Suitable collagen can be derived from a number of animal sources, either in the form of a solution or in the form of a dispersion. In one embodiment, the invention relates to the use of composite materials formed by intimately contacting collagen with a mucopolysaccharide under conditions at which they form a reaction product and subsequently covalently crosslinking the reaction product. Suitable mucopolysaccharides include, but are not limited to chondroitin 4-sulfate, chondroitin 6-sulfate, heparan sulfate, dermatan sulfate, keratan sulfate, heparin and hyaluronic acid.

Covalent crosslinking can be achieved by chemical, radiation, dehydrothermal or other covalent crosslinking techniques. A suitable chemical technique is aldehyde crosslinking, but other chemical crosslinking reactants are equally suitable. Dehydrothermal crosslinking, which is preferred, is achieved by reducing the moisture level of the composites to a very low level, such as by subjecting the composite material to elevated temperatures and high vacuum. Dehydrothermal crosslinking eliminates the necessity to add, and in the case of toxic materials such as aldehydes, to remove unreacted crosslinking agents; dehydrothermal crosslinking also produces composite materials containing a wider range of mucopolysaccharide content than is achieved with some chemical crosslinking techniques. The products of such syntheses are collagen molecules or collagen fibrils with long mucopolysaccharide chains attached to them.

Materials other than collagen could probably be contacted with chondroitin 6-sulfate and other mucopolysaccharides to yield blood-compatible materials. Such materials could include synthetic polymers such as the segmented polyurethanes, polyhydroxyethyl methacrylate and other "hydrogels", silicones, polyethylene terephthalate and polytetrafluoroethylene or modified natural polymers such as cellulose acetate or natural polymers such as elastin (the fibrous, insoluble, non-collagenous protein found in connective tissues such as the thoracic aorta and ligamentum nuchae) or pyrolytic carbon and other carbons which may have been treated thermally or by an electric arc. Such composites could be formed either by intimate mixing of the powdered solids or mixing of compatible solutions or dispersions of the two components or by coating with a mucopolysaccharide one of the materials mentioned in this paragraph. Irrespective of the method used to contact the mucopolysaccharide with the other material, the two components could be covalently bonded to form a material from which the mucopolysaccharide cannot be dissolved or extracted by contact with mucopolysaccharide solvents such as aqueous electrolytic solutions. Covalent bonding could be effected by a radiation grafting copolymerization technique using, for example, .gamma.-radiation from a cobalt-60 source. In all such procedures, chondroitin 6-sulfate or other mucopolysaccharides which do not interfere with normal blood clotting if accidentally eluted out of the composite material during use are clearly preferred over heparin which strongly interferes with normal blood clotting.

It is also quite probable that blood-compatible materials could be prepared by bonding, using an adhesive, the crosslinked collagen-mucopolysaccharide composite in the form of a sheet, film, or other form onto a variety of substrates. Such substrates would include synthetic polymers such as the segmented polyurethanes, polyhydroxyethyl methacrylate and other "hydrogels", silicones, polyethylene terephthalate and polytetrafluoroethylene or modified natural polymers such as cellulose acetate or natural polymers such as elastin or pyrolytic carbon and other carbons which may have been treated thermally or by an electric arc or metals such as vitalium, titanium and various steels. A suitable adhesive would, for example, be a silicone rubber adhesive.

Further, the method for producing the product of the present invention must make use of steps that are recognized as effective for inactivating viral and prion contamination. This gives the product a very high safety level while eliminating the inflammatory response. That is, the method for producing the product of the invention provides a product that is substantially free of viruses and prions without being physiologically incompatible. The phrase "substantially free of viruses and prions" means that the product does not contain infection-effective amounts of viruses and prions.

In particular, the collagen devices of the present invention may be prepared by enzyme treatment, e.g., with ficin and/or pepsin for about 1 to 2 hours at a temperature of about 36.5°C to 37.5°C, an alkali treatment, *e.g*., with an aqueous solution of 5% sodium hydroxide and 20% sodium sulfate at a pH of about 13 to 14, at a temperature of about 25°C to 30°C for a period of about 35 to 48 hours, or physiologically compatible collagen which is substantially free of active viruses and prions can be obtained from transgenic animals bred for the purpose of synthesizing human collagen in a readily harvestible form. See, e.g., U.S. Pat. No. 5,667,839.

More specifically, the invention preferably comprises the use of collagen treated by a process sufficient to achieve at least a 4 log clearance of virus, more preferably at least a 6 log clearance of virus, and even more preferably at least an 8 log clearance of virus, as measured with a statistical confidence level of at least 95%. For example, if the concentration of virus before treatment is 10⁷ and after treatment is 10¹, then there has been an 6 log clearance of virus. In preparing the dural substitutes of the present invention, a collagen dispersion is first prepared in a manner well known in the art. One such preparation is taught in U.S. Pat. No. 3,157,524. Another suitable preparation of collagen is taught in U.S. Pat. No. 3,520,402. The product is preferably nonantigenic in addition to being noninfectious and physiologically compatible.

The matrix can include biocompatible and/or bioresorbable materials other than collagen, although collagen is most preferred. Additional suitable polymers include, *e*.*g*., biocompatible and/or bioresorbable lactides, glycolides, and copolymers thereof, polycaprolactones, polyethylene carbonate, tyrosine polycarbonates, tyrosine polyacids, and polyanhydrides. The molecular weight of the polymer is preferably about 5000 to about 500,000.

As used herein, the term "polymer" refers *inter alia* to polyalkyls, polyamino acids and polysaccharides. Additionally, for external or oral use, the polymer may be polyacrylic acid or carbopol. As used herein, the term "synthetic polymer" refers to polymers that are not naturally occurring and that are produced via chemical synthesis. As such, naturally occurring proteins such as collagen and naturally occurring polysaccharides such as hyaluronic acid are specifically excluded. Synthetic collagen, and synthetic hyaluronic acid, and their derivatives, are included. Synthetic polymers containing either nucleophilic or electrophilic groups are also referred to herein as "multifunctionally activated synthetic polymers". The term "multifunctionally activated" (or, simply, "activated") refers to synthetic polymers which have, or have been chemically modified to have, two or more nucleophilic or electrophilic groups which are capable of reacting with one another (*i*.*e*., the nucleophilic groups react with the electrophilic groups) to form covalent bonds. Types of multifunctionally activated synthetic polymers include difunctionally activated, tetrafunctionally activated, and star-branched polymers.

Derivatives of various polysaccharides, such as glycosaminoglycans, can additionally be incorporated into the compositions of the invention. Glycosaminoglycans that can be derivatized according to either or both of the aforementioned methods include the following: hyaluronic acid, chondroitin sulfate A, chondroitin sulfate B (dermatan sulfate), chondroitin sulfate C, chitin (can be derivatized to chitosan), keratan sulfate, keratosulfate, and heparin. Derivatization of glycosaminoglycans by deacetylation and/or desulfation and covalent binding of the resulting glycosaminoglycan derivatives with synthetic hydrophilic polymers are described in further detail in U.S. Pat. Nos. 6,534,591; 6,323,278; 6,166,130; 6,165,489; 6,051,648; 5,874,500; 5,800,541; 5,752,974; 5,643,464; 5,550,187; 5,510,121; 5,476,666; 5,475,052; and 5,470,911. Covalent binding of collagen to synthetic hydrophilic polymers is described in U.S. Pat. No. 5,162,430.

In general, collagen from any source may be used in the compositions of the invention; for example, collagen may be extracted and purified from human or other mammalian source, such as bovine or porcine corium and human placenta, or may be recombinantly or otherwise produced. The preparation of purified, substantially non-antigenic collagen in solution from bovine skin is well known in the art. See U.S. Pat. No. 5,428,022. The term "collagen" or "collagen material" as used herein refers to all forms of collagen, including those that have been processed or otherwise modified.

Collagen of any type, including, but not limited to, types I, II, III, IV, or any combination thereof, may be used in the compositions of the invention, although type I is generally preferred. Either atelopeptide or telopeptide-containing collagen may be used; however, when collagen from a xenogeneic source, such as bovine collagen, is used, atelopeptide collagen is generally preferred, because of its reduced immunogenicity compared to telopeptide-containing collagen.

Collagen that has not been previously crosslinked by methods such as heat, irradiation, or chemical crosslinking agents is preferred for use in the compositions of the invention, although previously crosslinked collagen may be used. Non-crosslinked atelopeptide fibrillar collagen is commercially available from Collagen Corporation (Palo Alto, Calif.) under the trademarks ZYDERM I COLLAGEN and ZYDERM II COLLAGEN, respectively. Glutaraldehyde crosslinked atelopeptide fibrillar collagen is commercially available from Collagen Corporation under-the trademark ZYPLAST COLLAGEN.

Once the CG matrix has been prepared, the cavity is readied for application of the covering. Areas of tissue within the cavity that have been destroyed or damaged are surgically removed to prevent it from interfering with the healing process. The CG matrix, preferably in sheet form, is randomly stuffed into the cavity in a manner that minimizes the entrapment of air pockets between the tissue and the matrix. The surgical opening is sutured or stapled closed using conventional techniques and then covered with a bandage.

After implantation of the CG lattice, blood vessels and cells from underlying healthy tissue begin the process of infiltration of the grafted CG matrix. "Infiltration", as defined herein, further refers to allowing a sufficient period of time for this migration of cells and blood vessels into the lattice.

The collagen lattice eventually is biodegraded by collagenase and other natural enzymes into non-toxic substances that are digested, utilized, or eliminated by normal bodily processes. The lattice must retain its structural integrity until an adequate number of cells have reproduced within the lattice to regenerate the lost or removed tissue. If the lattice is biodegraded more quickly than this, it will be liquified and rendered useless before the cavity has healed.

The biodegradation rate should be roughly equal to approximately 25 to 30 days. This does not mean that the entire lattice should be biodegraded within 30 days. Instead, it indicates that a significant amount of biodegradation should commence within about 30 days, although remnants of the lattice may persist for several months or more. Routine experimentation by persons skilled in the art might indicate that this biodegradation rate should be modified somewhat for lattices that are seeded with cells, or for lattices that are used for purposes other than synthetic skin.

The biodegradation rate of a collagen lattice may be decreased (i.e., the lattice will endure for a longer period of time after grafting onto a wound) by increasing the collagen crosslinking density, by increasing the content of GAG that is crosslinked with collagen, or by decreasing the porosity of the lattice.

The lattice device should be sufficiently tough and strong to withstand suturing without tearing, and to prevent or limit tearing if subjected to accidental stresses caused by bandaging or medical operations or by patient movement. The two most important indices of strength of a lattice are tensile strength (which measures how much force is required to pull apart a specimen with a known cross-sectional area) and fracture energy (which measures how much work is required to create a tear of a given size).The strength of the lattice may be increased by increasing the crosslinking density or by decreasing the porosity of the lattice. The synthetic collagen lattice should resemble the collagen matrix that exists naturally within the type of tissue that is to be regenerated. This spatial arrangement will promote the growth of cells in orderly patterns that resemble undamaged tissue, thereby reducing scarring and promoting proper functioning of the regenerated tissue.

Another aspect of the present invention refers the filling of a surgical cavity or defect in a human or animal using seeded CG matrices. "Seeded CG matrices" refer to CG matrices into which cells (preferably harvested from a wound free site on the patient's body) have been introduced. Each cell that survives the seeding process can reproduce and multiply, thereby hastening the formation of a cavity-filling tissue. Preferred cells are adipocytes (fat cells) and/or preadipocytes (fat cell precursors that differentiate into adipocytes) derived from fat storage areas in the body or fat cells derived from bone marrow, stem cells, or mesenchymal cells that are harvested and developed under laboratory conditions and seeded onto the matrix prior to implantation. Seeded CG matrices are described in U.S. Pat. No. 4,060,081, the teachings of which are incorporated herein by reference in its entirety. Matrices that have been seeded are referred to as "cellular" while unseeded matrices are referred to as "acellular".

Seeded CG matrices may be autologous, i.e. matrices seeded with cells obtained from the human or animal having the bum or wound, or they may be heterologous, i.e. seeded with cells obtained from a donor. In addition, cells being used to seed a CG matrix may undergo genetic manipulation in order to prevent rejection or to change the cell's phenotype in some beneficial manner. Genetic manipulation includes introducing genetic matter into the cells so that the protein gene product or products are expressed in sufficient quantities to cause the desired change in phenotype. An example of suitable genetic matter includes the gene encoding for a growth factor along with the requisite control elements.

Implants of the invention may also include radio-opaque materials or radio-opaque elements, so that the biopsy site may be detected both with ultrasound and with X-ray or other radiographic imaging techniques. Radiopaque materials and markers may include metal objects such as clips, bands, strips, coils, and other objects made from radiopaque metals and metal alloys, and may also include powders or particulate masses of radiopaque materials. Radiopaque markers may be of any suitable shape or size, and are typically formed in a recognizable shape not naturally found within a patient's body, such as a star, square, rectangular, geometric, gamma, letter, coil or loop shape. Suitable radiopaque materials include stainless steel, platinum, gold, iridium, tantalum, tungsten, silver, rhodium, nickel, bismuth, other radiopaque metals, alloys and oxides of these metals, barium salts, iodine salts, iodinated materials, and combinations of these.

In addition, the implant of the invention may also include MRI-detectable materials or markers, so that the biopsy site may be detected both with ultrasound and with MRI or other imaging techniques. MRI contrast agents such as gadolinium and gadolinium compounds, for example, are suitable for use with ultrasound-detectable biopsy marker materials embodying features of the invention. Colorants, such as dyes (e.g., methylene blue and carbon black) and pigments (e.g., barium sulfate), may also be included in ultrasound-detectable biopsy marker materials embodying features of the invention.

Although this invention has been described in connection with its most preferred embodiment, additional embodiments are within the scope and spirit of the claimed invention. The preferred device of this invention is intended merely to illustrate the invention, and not limit the scope of the invention as it is defined in the claims that follow.

**Experimental Results.** Three animal studies with acellular scaffolds and two in vitro studies with preadipocytes are conducted using collagen glycosaminoglycan material and are detailed below.

### Study A:

The biocompatibility of two scaffolds is investigated in a porcine model. The sample materials, designated H16 and IM by Integra, (blocks measuring 2.5 x 2.5 x 1.0 cm³) are placed in the mammary tissue of three pigs and evaluated at explant time periods of 7, 21, and 60 days. At seven days, histology reveals giant cell infiltration with minimal fibrous tissue invasion. Neutrophil and giant cell infiltration is ongoing at 21 days with moderate to significant fibrous tissue invasion. At 60 days, invasion of well-organized granulation tissue is observed. The histopathology findings are considered positive and indicated a need for follow-up studies with an extended duration.

### Study B:

An *in vivo* evaluation of the commercially-available Integra Life Sciences scaffold (without the silicone backing) as a subdermal defect filler is performed. Alloderm (LifeCell Corporation) is investigated as a control material in this study. Sheets (2 x 2 x 0.1cm³) and rolls (2 cm in length, 0.5 cm in diameter) of Integra and Alloderm are implanted subdermally over the ventral thoracic and abdominal regions of six pigs. Explant time periods for this study are 14, 42, and 180 days. The Integra material demonstrate acceptable biocompatibility in this study.

### Study C:

The same, commercially-available Integra scaffold being used in Study B is also investigated as an acellular breast defect filler in a second study. Sheets of the Integra material are fashioned into rolls, approximately 8 cm in length and 1 cm n diameter, and implanted in the mammary tissue of six pigs. There are eight implant sites per pig, six sites containing implants and two sites present as empty controls. Two pigs are sacrificed or biopsied at each of three explant periods (14, 42, 180 and 364 days).

From an overall biocompatibility standpoint, under the conditions of this porcine animal model at periods of 14, 72, 179 and 364 days, the Integra implants are considered to be acceptable in the intramammary location.

Implant site volume is maintained between days 14 and 42. Between days 42 and 179, the implants in this pilot study followed one of two paths depending on the animal in which they are implanted.

Beyond the subacute reactions seen at a few sites post-surgically, the tissue reactions within the Integra matrices are associated with the normal absorptive process of the material and are no greater than slight to moderate.

Site volume maintenance correlated with matrix absorption: as the material absorbed, the sites became smaller. Although the one site with total absorption at day 364 has a greater amount of fibrous connective tissue than the empty control sites at that period, the absolute amount of new tissue is considerably less than the volume of material originally introduced into the site.

The degree of calcification of unabsorbed matrix appears to increase with in vivo residence.

### Study D:

An *in vitro* study of preadipocyte-seeded Integra material is initiated. The objectives of this study are to qualitatively assess the cytocompatibility of the scaffolds and to determine the appropriate preadipocyte seeding densities for a follow-up *in vivo* study. Cells are isolated from the epididymal fat pads of male Lewis rates and seeded on scaffold samples (1.0 x 1.0 x 0.1 cm³).

Cell proliferation and scaffold invasion are observed for up to two weeks using fluorescence microscopy and SEM. All materials are determined to be preadipocyte-compatible, facilitating cell growth and matrix infiltration. A fourfold increase in cell number is measured between week 1 and 2. The materials are more favorable for survival and growth of adipocytes than previously tested biomaterials.

### Study E:

Six scaffold materials, including the Integra product (identified as Avagen below), are evaluated for *in vitro* biocompatibility with human preadipocytes. All of these scaffolds are seeded with human subcutaneous preadipocytes at 5X10³ cell/cm² 2.5X10⁴ cells/cm² and 2.5X10⁵ cells/cm². A fluorometric assay is used to measures the total DNA in the scaffolds which indirectly estimates the number of cells. Proliferation of preadipocytes is observed with all scaffolds and is dependent on the initial seeding density and type of scaffold. A decrease in cell number and leptin secretion is observed in non-woven materials following adipocyte differentiation that may be due to the relatively rapid degradation rate of vicryl and the reduction in pH value which had an effect on pre-adipocyte biological activity. Hematoxylin and eosin (H&E) staining reveals the penetration of preadipocytes through the scaffolds after a three-week incubation. However, fluorescence labeling of adipocytes with Nile Red and histological staining with oil red O reveals that most of the mature adipocytes is on the surface of the scaffolds. Pre-adipocytes proliferated and differentiate well on both CBC foams and Avagen.™

In addition, information regarding procedural or other details supplementary to those set forth herein is described in cited references specifically incorporated herein by reference.

It would be obvious to those skilled in the art that modifications or variations may be made to the preferred embodiment described herein without departing from the novel teachings of the present invention. All such modifications and variations are intended to be incorporated herein and within the scope of the claims.

## Claims

1. A bioabsorbable soft tissue implant material for filling and closing soft-tissue cavities the implant material comprising a biologically-compatible in-growth matrix having interstices therein, wherein the interstices have dimensions effective to permit soft tissue to grow therein and wherein the matrix comprises a crosslinked collagen-glycosaminoglycan composite containing at least about 0.5% by weight glycosaminoglycan.

2. The implant of claim 1 wherein the interstices comprise between about 40 and about 60 percent of the implant material.

3. The implant of claim 1 wherein the interstices have a size of less than about 100 microns.

4. The implant of claim 1 wherein the collagen comprises between about 30% and about 94% of the implant material by weight.

5. The implant of claim 4 wherein the glycosaminoglycan is present in an amount sufficient to provide between about 6 percent and about 15 percent, by weight, of the collagen-glycosaminoglycan product.

6. The implant of claim 5 wherein the glycosaminoglycan is selected from the group consisting of hyaluronic acid, chondroitin 6-sulfate, chondroitin 4-sulfate, heparin, heparan sulfate, keratan sulfate, dermatan sulfate, chitin, and chitosan.

7. The implant of claim 5 wherein the glycosaminoglycan is chondroitin 6-sulfate.

8. The implant of claim 1, wherein the implant self expands to conform to the tissue void when in contact with body fluid.

9. The implant of claim 1, wherein the implant is of any geometrical shape.

10. The implant of claim 1, wherein the implant is a sheet having an overall thickness of from about 25 to about 100 mils.
